# EUROPEAN PATENT APPLICATION

(11) **EP 4 559 395 A1**
(43) Date of publication of application: **28.05.2025**
(21) Application number: 23211911.5
(22) Date of filing: 24.11.2023
(51) Int. Cl.: A61B 5/319, A61B 5/327, A61B 5/00

(54) **PHYSIOLOGICAL SIGNAL SYNTHESIZING**

(71) Applicant: Koninklijke Philips N.V., 5656 AG Eindhoven (NL)
(72) Inventor: VERHAEGH, Wilhelmus Franciscus Johannes, Eindhoven (NL); PEREVERZEVA, Anna, Eindhoven (NL); BONOMI, Alberto Giovanni, 5656AG Eindhoven (NL)
(74) Representative: Philips Intellectual Property & Standards

(57) **Abstract**

Proposed concepts thus aim to provide schemes, solutions, concept, designs, methods and systems pertaining to synthesizing a physiological signal. In particular, embodiments aim to provide a method for synthesizing a physiological signal by generating a signal template and separately generating a plurality of individual waves before combining the two. In other words, it is proposed that by separating the generation of a signal template (i.e., a pattern / rhythm for the physiological signal to-be-generated) and the generation of individual waves (i.e., of which the physiological signal to-be-generated will be formed out of), different and more optimal methods for generating each component can be used respectively.

## Description

### FIELD OF THE INVENTION

This invention relates to the field of physiological signals, and more specifically, to the field of synthesizing a physiological signal.

### BACKGROUND OF THE INVENTION

Synthetic data is information that is artificially manufactured rather than generated by real-world events. It is created algorithmically and can be used, for example, as a stand-in for test data sets of production or operational data, to validate mathematical models, and to train machine-learning (ML) models. While gathering high-quality data from the real world is difficult, expensive and timeconsuming, synthetic data technology enables users to quickly, easily, and digitally generate the data in whatever amount they desire while also customizing to their specific needs.

Especially for health care related application areas, gathering large collections of data to train and test ML algorithms can be very challenging, if not impossible. Not only do privacy regulations make it difficult to obtain sufficient data with corresponding annotations, but certain sub-populations of patients may be underrepresented in data studies, or data may be biased. Synthetic data that reproduces the statistical properties of real data and has comparable predictive power has the potential to address these problems, thereby improving software and system testing, and speeding up ML training and testing.

### SUMMARY OF THE INVENTION

The invention is defined by the claims.

According to examples in accordance with an aspect of the invention, there is provided a method for synthesizing a physiological signal.

The method comprises: generating a signal template for a physiological signal of a first type using a predetermined physiological model; generating a plurality of individual waves for a physiological signal of the first type; and generating a physiological signal of the first type using the generated signal template and the generated plurality of individual waves.

Proposed concepts thus aim to provide schemes, solutions, concepts, designs, methods and systems pertaining to synthesizing a physiological signal. In particular, embodiments aim to provide a method for synthesizing a physiological signal by generating a signal template and separately generating a plurality of individual waves before combining the two.

In other words, it is proposed that by separating the generation of a signal template (i.e., a pattern / rhythm for the physiological signal to-be-generated) and the generation of individual waves (which the physiological signal to-be-generated will be formed out of), different and more optimal methods for generating each component can be used respectively. For example, the signal template is generated using a predetermined physiological model such that the template can be guaranteed to stay within physiological limits, whereas the plurality of individual waves may be generated using a neural network such that the individual waves may be more realistic.

The inventors have therefore realized that by combining two approaches (one for the signal template and one for the plurality of individual waves), the benefits of two approaches can be combined. For example, more realistic physiological signals can be synthesized, generating near-real waves while avoiding privacy concerns, not being limited to real data, and still having significant control over certain parameters of said signal (by separately generating the signal template).

For example, the signal template can be generated in a manner that ensures the signal to-be-generated stays within physiological limits (i.e., using a predetermined physiological model) while also providing a high level of control over signal parameters to a user, such that the signal to-be-generated can fit the user's expectations/needs. In contrast, the plurality of individual waves can be generated in a manner that ensures realism (i.e., including realistic noise such that signal to-be-generated will look like a signal observed in the real world). Approaches which provide realism, such as GANs, however, may not allow for a high level of control while also not guaranteeing that the results stay within physiological limits. These downsides, however, can be greatly limited by restricting them to individual waves rather than the entire signal to-be-generated. The individual waves can then ultimately be combined according to the signal template to provide an overall synthesized signal which is guaranteed to stay within physiological limits while being composed of highly-realistic parts.

In a simple example, considering the synthetization of an ECG signal, the signal template may dictate the order and rhythm of different wave types such as P-waves, QRS-waves and T-waves, etc. while also allowing for the inclusion of arrythmias or ectopic beats. The plurality of individual waves being generated may thus contain P-waves, QRS-waves and T-waves, etc., such that these individual waves can be combined (e.g., joined together / merged) according to the generated signal template. In this way, an ECG signal is ultimately synthesized, the parameters of which, for example, are able to be controlled by a user while still providing a realistic output.

This invention may be of particular use in the generation of synthetic data to train and test machine-learning models. For example, this method can facilitate the generation of synthetic data which, while avoiding privacy concerns, can be tailored to a user's specific needs (via the generation of the signal template) while also providing highly realistic results (via the separate generation of the individual waves).

Ultimately, an improved method for synthesizing a physiological signal is provided.

In some embodiments, generating the plurality of individual waves may be responsive to the generated signal template, such that the plurality of individual waves is generated to fit the generated signal template. In this way, the plurality of individual waves can be generated specifically to fit the signal template. This therefore increases the effectiveness and/or the efficiency of the method such that individual waves are not generated that are not needed while also ensuring that the individual waves will be able to fit into (or combine together according to) the signal template as required.

In some embodiments, generating the plurality of individual waves may comprise generating timing data for each of the plurality of individual waves. For example, this timing data provides an effective and/or efficient method of 'telling' the individual waves how they should be arranged in relation to one another and/or in relation to the final synthesized wave, i.e., their placement and/or duration. For example, the timing data may tell one of the individual waves to start (in the physiological signal to-be-generated) at 0 seconds and last 1 second, another of the individual waves to start at 1.5 seconds and last 0.5 seconds, and another of the individual waves to start at 2 seconds, etc.

In some embodiments, generating the physiological signal of the first type may comprise combining at least two individual waves of the plurality of individual waves together according to the signal template. For instance, two of the individual waves may be joined together (i.e., end to end) or may partially overlap in time, in which case the waves may be combined / added up.

In some embodiments, the plurality of individual waves may be generated using a first machine-learning model. In this way, a more efficient and/or effective method of generating realistic individual physiological waves can be provided.

In some embodiments, the first machine-learning model may comprise a generative adversarial network, GAN, or a variational autoencoder, VAE. These have been found to be beneficial forms of machine-learning model for generating realistic physiological signals.

In some embodiments, the physiological signal of a first type may comprise an ECG signal. Synthesizing ECG signals facilitates the generation of synthetic ECG datasets for the training and/or testing of machine-learning models configured to, for example, analyze ECG signals.

In some embodiments, the generated signal template may comprise at least one arrhythmia. This allows ECG signals to be synthesized which include arrhythmia which may ultimately provide more useful training and/or testing datasets.

In some embodiments, the arrhythmia may comprise an ectopic beat. This allows ECG signals to be synthesized which include ectopic beats which may ultimately provide more useful training and/or testing datasets.

In some embodiments, the plurality of individual waves may comprise individual waves of at least two of the following types: P-waves; QRS-waves; T-waves; and/or f-waves. These are types of waves typically present in an ECG signal.

In some embodiments, the physiological signal of a first type may comprise at least one of: an SpO2 signal; an EMG signal; an EEG signal; a respiration rate signal; and/or a haemoglobin levels signal. These are all useful physiological signals to synthesize.

In some embodiments, the generated signal template may comprise a pattern of a first rhythm. This allows the synthetization of a physiological signal with a pattern of a first rhythm.

In some embodiments, the generated signal template may comprise a pattern of a second rhythm different to the first rhythm. This allows the synthetization of a physiological signal comprising patterns of at least two different rhythms.

In some embodiments, the method may further comprise obtaining a user input describing at least one desired physiological parameter for the physiological signal; wherein generating the signal template is responsive to the user input. In this way, a user may specify physiological parameters for the physiological signal to-be-synthesized, for example, a particular rhythm (e.g., heart rate or respiration rate) or the types of waves to be included.

In some embodiments, the generated signal template may comprise a pattern of at least two different types of waves. This allows the synthetization of a physiological signal comprising patterns of at least two different types of waves.

According to another aspect of the invention, there is provided a computer program comprising code means for implementing the method of any herein disclosed method when said program is run on a processing system.

According to another aspect of the invention, there is provided a system for synthesizing a physiological signal. The system comprising a processor configured to: generate a signal template for a physiological signal of a first type using a predetermined physiological model; generate a plurality of individual waves for a physiological signal of the first type; and generate a physiological signal of the first type using the generated signal template and the generated plurality of individual waves.

According to another aspect of the invention, there is provided a machine-learning training data generation system comprising a system for synthesizing a physiological signal according to any herein-described embodiment.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for synthesizing a physiological signal according to a proposed embodiment;
Fig. 2A is a diagram depicting an example signal template according to a proposed embodiment;
Fig. 2B is a diagram depicting an example physiological signal generated according to the example signal template of Fig. 2A;
Fig. 3 is a flow diagram of a method for synthesizing a physiological signal according to a proposed embodiment;
Fig. 4 is a simplified block diagram of a system for synthesizing a physiological signal according to a proposed embodiment;
Fig. 5 is a simplified block diagram of a machine-learning training data generation system comprising a system for synthesizing a physiological signal according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to synthesizing a physiological signal. According to proposed concepts, a number of possible solutions may be implemented separately or jointly. That is, although these possible solutions may be described below separately, two or more of these possible solutions may be implemented in one combination or another.

Embodiments of the invention aim to provide a method for synthesizing a physiological signal. This can be achieved by generating a signal template and separately generating a plurality of individual waves before combining the two.

In other words, it is proposed that by separating the generation of a signal template (i.e., a pattern / rhythm for the physiological signal to-be-generated) and the generation of individual waves (which the physiological signal to-be-generated will be formed out of), different and more optimal methods for generating each component can be used respectively. For example, the signal template is generated using a predetermined physiological model such that the template can be guaranteed to stay within physiological limits, whereas the plurality of individual waves may be generated using a neural network such that the individual waves may be more realistic.

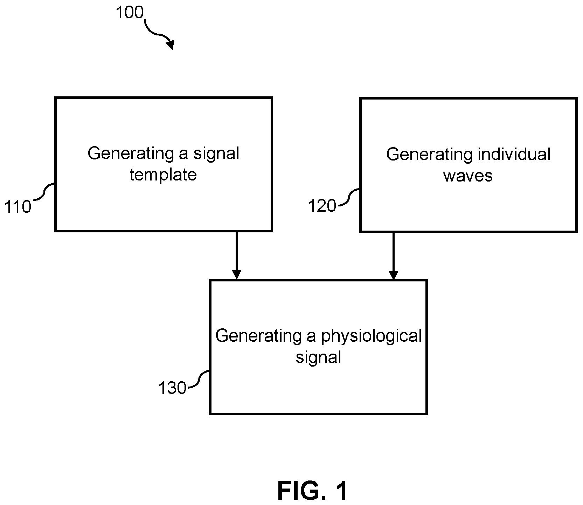

Referring now to Fig. 1, there is depicted a simplified flow diagram of a method 100 for synthesizing a physiological signal according to a proposed embodiment.

The method 100 begins with the step 110 of generating a signal template for a physiological signal of a first type using a predetermined physiological model. `Using a predetermined physiological model' can also be understood as `based on a predetermined physiological model'. For example, in some embodiments, the predetermined physiological model can comprise one or more Markov models which take into account known physiological data. In other embodiments, for example, any suitable form of probabilistic model can be used to generate a signal template, e.g., a rhythm sequence.

In this embodiment, the physiological signal of a first type comprises at least one of: an SpO2 signal; an EMG signal; an EEG signal; a respiration rate signal; and/or a haemoglobin levels signal. These are all useful physiological signals to synthesize. In other embodiments, however, the physiological signal of a first type can comprise any suitable physiological signal, such as an ECG signal, and the concepts described herein are not limited to any particular physiological signal.

In this embodiment, the generated signal template comprises a pattern of a first rhythm. This allows the synthetization of a physiological signal with a pattern of a first rhythm. This is not essential to the working of the invention however, and in other embodiments, the signal template can comprise a pattern without any particular rhythm. In this embodiment, however, the generated signal template further comprises a pattern of a second rhythm different to the first rhythm. This allows the synthetization of a physiological signal comprising patterns of at least two different rhythms, e.g., including a change of rhythm or a physiological rate, or an arrythmia.

It should be noted that the generated signal template can indicate not only a rhythm of individual waves but also the types of the individual waves to be placed within the rhythm. In other words, a pattern of the generated signal template can indicate not only a rhythm of the individual waves but the type of individual waves that should make up said rhythm too. For instance, in a simple example, a pattern of a generated signal template can indicate that a first type of wave should be followed a second type of wave and that this pattern should repeat every three seconds.

In some embodiments, a rhythm can comprise a physiological rate such as a heart rate or a respiration rate, dependent on the type of physiological signal being synthesized. In some embodiments, the generated signal template can comprise a pattern of at least two different types of waves. This allows the synthetization of a physiological signal comprising patterns of at least two different types of waves.

In step 120, a plurality of individual waves is generated for a physiological signal of the first type. For example, the generated plurality of individual waves could take the form of a library/database of generated individual waves for a physiological signal of the first type from which individual waves can be taken and used to generate the physiological signal of the first type (in step 130) according to the generated signal template.

In this embodiment, the plurality of individual waves is generated using a first machine-learning model which comprises a variational autoencoder, VAE. This has been found to be a beneficial form of machine-learning model for generating realistic physiological signals. In other embodiments, however, the plurality of individual waves can be generated in any suitable way such as, for example, using a neural network or a method which does not comprise machine-learning models at all.

In some embodiments, two or more of the plurality of individual waves can be generated together, i.e., already combined. For instance, an A-type individual wave can be generated along with, and already connected to, a B-type individual wave. If a signal template indicates, for example, the presence of an A-type wave followed by a B-type wave, the joint unit from the plurality of individual waves can thus be used.

In step 130, a physiological signal of the first type is generated using the generated signal template and the generated plurality of individual waves. `Using the generated signal template and plurality of individual waves' can also be understood as `based on the generated signal template and plurality of individual waves'.

Synthetic signals can be generated using various techniques. It is established that models of physical systems can be used to simulate physiological behavior and thus generate signals. These can be models based on physical equations, differential equations, but also probabilistic models can be used. Furthermore, physiological models of diseases or organs can be addressed in this way. Another branch of synthetic data generation is the use of pure data driven approaches, such as generative adversarial networks (GANs), variational autoencoders (VAE) or diffusion models, which are machine-learning frameworks which can be used to generate data resembling real-life example data.

In the present invention, it is thus essentially proposed to use a combined approach in which artificial generators are used to generate physiological data and a model to generate a physiological signal template. The main elements of the invention can therefore be understood as, for example, a computer model to generate rhythms and patterns to switch between different rhythms (for example to generate heart rhythms and insert ectopic beats); a data driven generator to generate individual waves (for example, multiple data driven generators for different types of waves respectively); and a framework to stitch together the individual waves according to the generated rhythms. This allows the generation of synthetic data, which can have value in itself, but can also be used to generate synthetic data sets that have value and can be used to train and/or test machine-learning models.

In some embodiments, steps 120 and 130 can essentially be combined. For example, in some embodiments, the signal template generated in step 110 can be used to generate a sequence of individual waves that are already connected together, such that the physiological signal of the first type is generated in a single step after the generation of the signal template. For example, in some embodiments, generative Long-Short-Term-Memory networks can be used which are provided the signal template as an input and then generate the required sequence of individual waves in one go in such a way that they are already connected to each other, and the physiological signal thus synthesized. This can thus be understood as generating a connected sequence of one or more beats (individual waves) using the signal template that will already be connected according to the template. For example, in some embodiments, if the physiological signal comprises an ECG wave, all the waves for an AF episode can be generated together, all the waves for an SR episode generated together, and then these episode-wave-sequences subsequently connected together to synthesize the physiological signal.

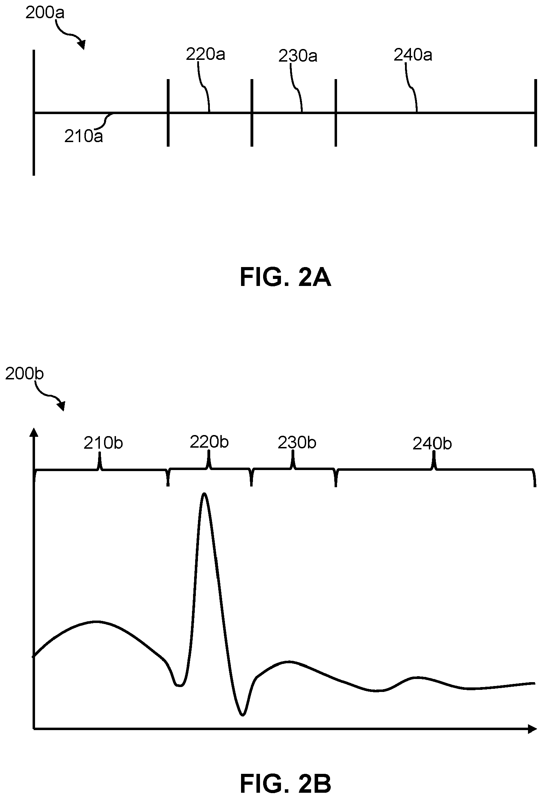

Referring now to Fig. 2A, there is depicted a diagram of an example signal template 200a according to a proposed embodiment. For example, this signal template 200a is for an ECG signal and comprises a pattern made up of a space for a P-wave 210a; a space for a QRS-wave/complex 220a; a space for a T-wave 230a; and a space for f-waves 240a. This signal template 200a therefore includes timing data as it describes where each wave should be placed and the duration of each wave, as well as specifying the type of wave to be placed in each section. The signal template 200a could then further indicate this pattern repeating with a certain rhythm, and possibly further including different rhythms and/or arrythmias such as ectopic beats.

Referring now to Fig. 2B, there is depicted a diagram of an example physiological signal 200b according to the example signal template 200a of Fig. 2A. For instance, it can be seen how the spaces indicated in the signal template 200a correspond to the placement of individual waves in the final synthesized physiological (ECG) signal 200b. For instance, a generated P-wave 210b is placed in its corresponding space 210a; a generated QRS-wave/complex 220b is placed in its corresponding space 210b; a generated T-wave 230b is placed in is corresponding space 210c; and generated f-waves 240b are placed in their corresponding space 240a. Where the waves connect to one another, the individual waves can be joined together, such that the end of one wave joins to the end of the other wave, or, if the placement of the individual waves overlap, the individual waves can be combined, added, or summed together where they overlap. In this way, a single, coherent physiological signal can be generated from a plurality of individual waves and a signal template.

For example, during atrial fibrillation (AF), a signal template could be generated which repeatedly has QRS- and T-waves (but no P-waves), and the generated f-waves could then be overlayed on top of all of them during the entire AF episode. In other words, in some embodiments, the signal template can comprise more than one type of wave at a time, in which case, the final synthesized wave can comprise a combination/summation of the two or more types of simultaneous individual waves.

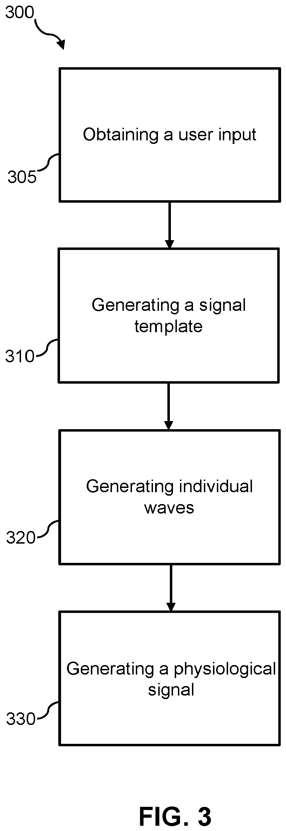

Referring now to Fig. 3, there is depicted a flow diagram of a method 300 for synthesizing a physiological signal according to a proposed embodiment.

In step 305, a user input is obtained describing at least one desired physiological signal parameter for the physiological signal.

In step 310, a signal template for a physiological signal of a first type is generated using a predetermined physiological model. In this embodiment, the generation of the signal template is also responsive to the user input, i.e., generating the signal template is further based on the user input. In this way, a user can specify physiological parameters for the physiological signal to-be-synthesized, for example, a particular rhythm (e.g., heart rate or respiration rate depending on the type of physiological signal to be synthesized) or the types of waves to be included. User input is not essential for the working of the invention, however, and in some embodiments, step 305 can be omitted.

In this embodiment, the physiological signal of a first type comprises an ECG signal, for example, a 12-lead ECG signal. Synthesizing ECG signals facilitates the generation of synthetic ECG datasets for the training and/or testing of machine-learning models configured to, for example, analyze ECG signals. As described in relation to method 100 of Fig. 1, in other embodiments, the physiological signal of a first type can comprise any suitable physiological signal.

Further, in this embodiment, the generated signal template comprises at least one arrhythmia. This allows ECG signals to be synthesized which include arrhythmia which may ultimately provide more useful training and/or testing datasets. In fact, in this embodiment, the arrhythmia comprises an ectopic beat. This allows ECG signals to be synthesized which include ectopic beats which may ultimately provide more useful training and/or testing datasets. The inclusions of arrhythmia and/or ectopic beats are not essential for the working of the invention, however.

For example, in embodiments where the physiological signal comprises an ECG signal, the predetermined physiological model can comprise two Markov models for rhythm switching. One model, with only atrial fibrillation (AF) and sinus rhythm (SR), can be used to generate a scheme of successive SR and AF episodes. The other model, also with only two states, can be used, for example, to insert atrial ectopic beats outside the AF episodes. In another example, a more generalized Markov model can be used as the predetermined physiological model. For example, the Markov model can comprise the following components: an SR component; an atrial premature beats (APB) component; an interpolated PVC beats component; an AF component; and at least two PVC components. Parameters for the transition probabilities between states can be controlled to influence the length of SR, AF and PVC episodes, and the rate at which ectopic beats (e.g., APB and interpolated PVC beats) occur. For PVC episodes, the sequence of the at least two PVC components (nodes) can be set to match a desired repetitive pattern (e.g., a pattern can be used to generate a sequence of two nodes, PVC0 and PVC 1, with an annotation that PVC0 is a ventrical beat, and PVC1 is a normal beat). The skilled person would understand without any undue burden how Markov models can be used in a variety of implementations to generate signal templates according to the present invention. Markov models similar to the one described above allow for easy extension of the kind of rhythms to switch between.

For example, it is noted that the Markov model including at least two PVC components allows bigeminy patterns to be generated, the Markov model including at least three PVC components allows trigeminy patterns to be generated, such as a VNN pattern, and the Markov model including at least four PVC components allows quadrigeminy patterns to be generated.

In step 320, responsive to the signal template generated in step 310, a plurality of individual waves is generated such that the plurality of individual waves is generated to fit the generated signal template. `Responsive to the signal template' can also be understood as `based on the generated signal template'. In this way, the plurality of individual waves can be generated specifically to fit the signal template. This therefore increases the effectiveness and/or the efficiency of the method such that individual waves are not generated that are not needed while also ensuring that the individual waves will be able to fit into (or combine together according to) the signal template as required. For example, if the signal template indicates that it needs a wave type A that is 2 seconds long, a wave type B that is 1 second long, and another wave type A that is 1 second long, these individual waves can accordingly be generated. This feature is not essential for the working of the invention, however, as described in relation to step 120 of method 100.

In this embodiment, generating the plurality of individual waves also comprises generating timing data for each of the plurality of individual waves. For example, this timing data provides an effective and/or efficient method of 'telling' the individual waves how they should be arranged in relation to one another and/or in relation to the final synthesized wave, i.e., their placement and duration. For example, the timing data can tell one of the individual waves to start (in the physiological signal to-be-generated) at 0 seconds and last 1 second, another of the individual waves to start at 1.5 seconds and last 0.5 seconds, and another of the individual waves to start at 2 seconds, etc. For example, the timing data can indicate the position of an R peak in a QRS-wave(complex), such that this info can be taken into account during the generation of the synthetic wave such that the QRS-wave will be placed at the correct time-position. The generation of timing data is not essential for the working of the invention, however, and in other embodiments, external timing data can be used to arrange the individual waves according to the generated signal template.

In another embodiment, the signal template can comprise timing data such that the timing data can be used during generation of the individual waves, for example, to tell a wave generator to generate a QRS-wave with the R peak at 0.3 seconds after the start. For example, for sinus rhythm, parasympathetic stimulation (respiratory) and baroreflex regulation can be modelled by two components in a frequency spectrum, meaning that generated heart rhythms (RR intervals) can vary over time due to respiration and so-called Mayer waves. This can thus be used to inform the generation of timing data. For atrial fibrillation, for example, an atrioventricular (AV) node model can be used to generate RR intervals, where ventricals can be assumed to be activated by atrial impulses arriving to the AV node corresponding to a Poisson process, and where some refractory period can be taken into account. Again, this can thus be used to inform the generation of timing data.

In this embodiment, the plurality of individual waves comprise individual waves of at least two of the following types: P-waves; QRS-waves; T-waves; and/or F-waves. These are types of waves typically present in an ECG signal. In other embodiments, however, the plurality of individual waves can comprise waves of any suitable wave types. For example, in other embodiments, the plurality of individual waves can further comprise individual waves of at least one of the following types: ventricular complex; torsades de pointes; U-waves; and/or noise.

Further, in this embodiment, the plurality of individual waves is generated using a first machine-learning model comprising a generative adversarial network, GAN. This has been found to be a beneficial form of machine-learning model for generating realistic physiological signals. In other embodiments, however, the plurality of individual waves can be generated using any suitable method such as, for example, a neural network or a method which does not comprise machine-learning models at all.

In this embodiment, the different types of waves are all generated using the same machine-learning model, but in other embodiments, the different types of waves can be generated using different machine-learning models, for instance each type of wave can be generated using its own respective machine-learning model (e.g., its own respective GAN).

In step 330, a physiological signal of the first type (an ECG signal) is generated/synthesized using the generated signal template of step 310 and the generated plurality of individual waves of step 320. `Using the generated signal template and plurality of individual waves' can also be understood as `based on the generated signal template and plurality of individual waves'.

In this embodiment, generating the physiological signal of the first type comprises combining at least two individual waves of the plurality of individual waves together according to the signal template. For instance, two of the individual waves can be joined together (i.e., end to end) or can partially overlap in time, in which case the waves may be combined/summed/added up. In other embodiments, however, the individual waves can be placed separately from one another and the gaps between them filled in using any suitable method to generate the physiological signal.

In an example, for synthesizing an ECG signal, RR-intervals (i.e., the interval between successive R peaks) can be generated by a computer model or generator comprising a predetermined physiological model (for example, a different generator/model for atrial fibrillation, AF, and sinus rhythm, SR); individual QRST-waves and P-waves can be generated by a model or by a generator (ML model); and f-waves can be generated by a computer model or a generator. The QRST-, P- and f-waves can then be stitched together according to the scheme of RR-intervals generated in the first step.

By using a computer model to generate RR-intervals, and using generators for the QRST-, P-, and f-waves respectively, this method facilitates the generation of realistic output signals varying beyond what is given as input datasets, while on the other hand, being easily controllable to generate rhythms with desired properties.

In an example embodiment for synthesizing ECG signals, three different generators (machine-learning models) are used to generate PQRST-waves, f-waves, and noise respectively, that are each trained on corresponding sets of real data samples. The generator for the PQRST-waves generates not only the waves themselves but also timing information to use in the scheme/template, such as the start of the Q-wave, the position of the R-peak, the end of the S-wave, and the end of the T-wave (also known as fiducial points). Typically, after such a generator model has been trained, it is fed with random noise to generate various instances of the desired waves (i.e., the random noise plays the role of a seed from which the desired waves can grow/form).

In this example embodiment, the method can essentially be seen as including five stages. First, is the ventricular rhythm stage. This includes an AV-node model for the atrial fibrillation component and a respiratory rhythm model for the sinus rhythm component. Next, is the ventricular morphology stage. This includes generating QRST-waves from a generator for PQRST-waves for both the atrial fibrillation and sinus rhythm components. Next, is the atrial morphology stage. This includes generating f-waves for the atrial fibrillation component using an f-wave generator and generating P-waves for the sinus rhythm component using the PQRST-wave generator. Next, is the rhythm switching stage in which the atrial fibrillation and sinus rhythm components are fed into a Markov model which generates a synthetic ECG signal from the two components, and can additionally add atrial ectopic beats to the generated ECG. Finally, is the noise stage in which a noise generator is used to generate noise to add to the final synthetic ECG signal (to make the synthetic ECG signal more realistic).

In a second example embodiment, the rhythm generation component can be divided into atrial rhythm, ventricular rhythm, and an atrial-ventricular conduction pattern. The atrial rhythm would define the time at which a specific atrial electrical activity should take place and during which a P- or f-wave generated by the successive morphology components should be positioned. The ventricular rhythm selector would determine the temporal location of QRS morphology complexes, while the atrial-ventricular conduction pattern would act as a further input to suppress upcoming ventricular beats by mimicking the effect of an AV-block or ventricular dissociation. Once the location of atrial waves (P and f) and ventricular waves (QRST) are established over time, the morphology information can be superimposed on the base synthetic ECG signal.

Data generated using the herein disclosed embodiments of the invention can be used for training and testing of machine learning tools, for example, to segment sequences into different individual waves, to automatically determine where arrhythmias occur, etc. Other use cases can include testing accuracy of existing and new software for physiological signal interpretation and identifying stress cases for physiological signal interpretation algorithms. Using the proposed invention, it will be easier to combine and control various properties in a dataset to-be-generated while also obtaining realistic output sequences.

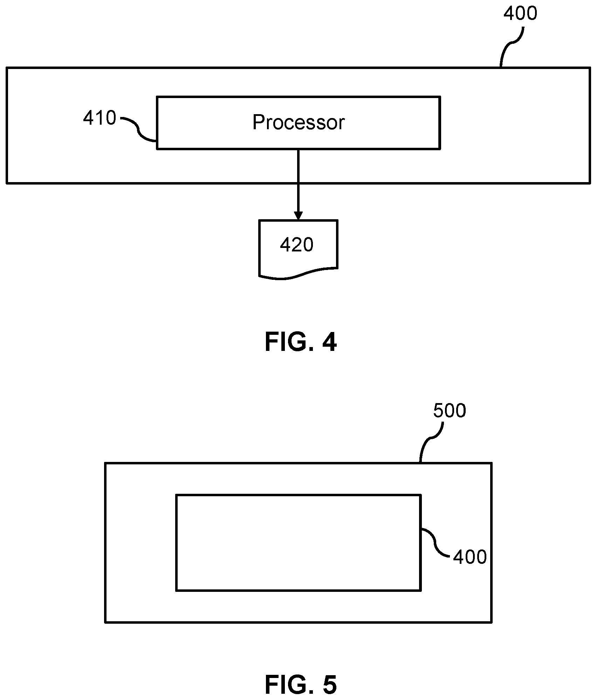

Referring now to Fig. 4, there is depicted a system 400 for synthesizing a physiological signal. The system comprises a processor 410 configured to: generate a signal template for a physiological signal of a first type using a predetermined physiological model; generate a plurality of individual waves for a physiological signal of a first type; and thus generate an output 420 using the generated signal template and the generated plurality of individual waves. The output 420 comprises a physiological signal of the first type.

In some embodiments, the processor 410 can comprise a machine-learning model. In some embodiments, the system 400 can further comprise an input interface 420 for obtaining, for example, user input or the predetermined physiological model.

Referring now to Fig. 5, there is depicted a machine-learning training data generation system 500 comprising a system 400 for synthesizing a physiological signal according to a proposed embodiment. For example, the machine-learning training data generation system 500 can be understood as a system for synthesizing a plurality of physiological signals (using system 400) such that a dataset for training and/or testing a machine-learning model can be generated.

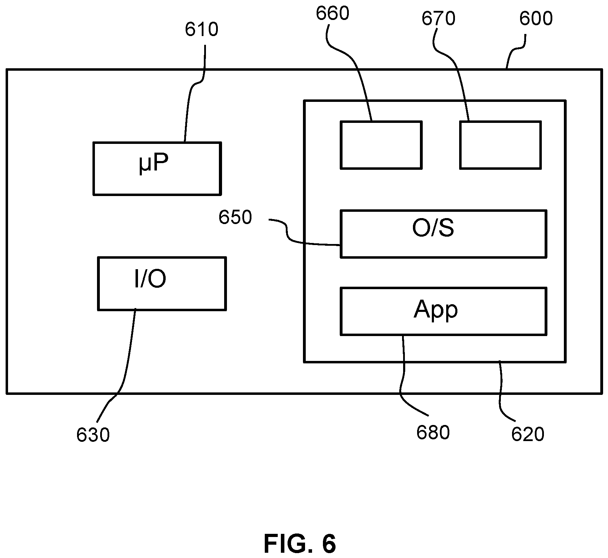

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 800 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1 and 3, and the systems of Figs. 4 and 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for synthesizing a physiological signal, the method comprising:
generating a signal template (110) for a physiological signal of a first type using a predetermined physiological model;
generating a plurality of individual waves (120) for a physiological signal of the first type; and
generating a physiological signal (130) of the first type using the generated signal template and the generated plurality of individual waves.

2. The computer-implemented method of claim 1, wherein generating the plurality of individual waves (320) is responsive to the generated signal template, such that the plurality of individual waves is generated to fit the generated signal template.

3. The computer-implemented method of claim 2, wherein generating the plurality of individual waves comprises generating timing data for each of the plurality of individual waves.

4. The method of any of claims 1 to 3, wherein generating the physiological signal (330) of the first type comprises combining at least two individual waves of the plurality of individual waves together according to the signal template.

5. The computer-implemented method of any of claims 1 to 4, wherein the plurality of individual waves is generated using a first machine-learning model, and preferably wherein the first machine-learning model comprises a generative adversarial network, GAN, or a variational autoencoder, VAE.

6. The computer-implemented method of any of claims 1 to 5, wherein the physiological signal of a first type comprises an ECG signal.

7. The computer-implemented method of claim 6, wherein the generated signal template comprises at least one arrythmia, and preferably wherein the arrhythmia comprises an ectopic beat.

8. The computer-implemented method of claim 6 or 7, wherein the plurality of individual waves comprises individual waves of at least two of the following types: P-waves; QRS-waves; T-waves; and/or f-waves.

9. The computer-implemented method of any of claims 1 to 5, wherein the physiological signal of a first type comprises at least one of: a blood pressure signal; an SpO2 signal; an EMG signal; an EEG signal; a respiration rate signal; and/or a haemoglobin levels signal.

10. The computer-implemented method of any of claims 1 to 9, wherein the generated signal template comprises a pattern of a first rhythm.

11. The computer-implemented method of claim 10, wherein the generated signal template comprises a pattern of a second rhythm different to the first rhythm.

12. The computer-implemented method of any of claims 1 to 11, wherein the method further comprises:
obtaining a user input (305) describing at least one desired physiological parameter for the physiological signal;
wherein generating the signal template (310) is responsive to the user input.

13. A computer program comprising code means for implementing the method of any of claims 1 to 12 when said program is run on a processing system.

14. A system (400) for synthesizing a physiological signal, the system comprising:
a processor (410) configured to:
generate a signal template for a physiological signal of a first type using a predetermined physiological model;
generate a plurality of individual waves for a physiological signal of the first type; and generate a physiological signal of the first type using the generated signal template and the generated plurality of individual waves.

15. A machine-learning training data generation system (500) comprising the system (400) of claim 14.
